(19) 

Europäisches
Patentamt
European
Patent Office
Office européen
des brevets

(11) **EP 2 652 708 B1**

(12) **EUROPEAN PATENT SPECIFICATION**

(45) Date of publication and mention
of the grant of the patent:
**28.01.2015 Bulletin 2015/05**

(21) Application number: **11801671.6**

(22) Date of filing: **09.12.2011**

(51) Int Cl.:
***G06T 11/00*** *(2006.01)*

(86) International application number:
**PCT/EP2011/072332**

(87) International publication number:
**WO 2012/080125 (21.06.2012 Gazette 2012/25)**

(54) **A METHOD AND A SYSTEM FOR IMAGE INTEGRATION USING CONSTRAINED OPTIMIZATION FOR PHASE CONTRAST IMAGING WITH AN ARRANGEMENT OF GRATINGS**

VERFAHREN UND SYSTEM ZUR BILDINTEGRATION MIT EINGESCHRÄNKTER OPTIMIERUNG FÜR PHASENKONTRASTBILDGEBUNG MIT EINER ANORDNUNG AUS GITTERN

PROCÉDÉ ET SYSTÈME PERMETTANT UNE INTÉGRATION D'IMAGE À L'AIDE D'UNE OPTIMISATION SOUS CONTRAINTES POUR UNE IMAGERIE PAR CONTRASTE DE PHASE AVEC UN AGENCEMENT DE RÉSEAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.12.2010 EP 10194726**

(43) Date of publication of application:
**23.10.2013 Bulletin 2013/43**

(73) Proprietor: **PAUL SCHERRER INSTITUT
5232 Villigen PSI (CH)**

(72) Inventors:
• **STAMPANONI, Marco
CH-5304 Endingen (CH)**

• **THÜRING, Thomas
CH-6214 Schenkon (CH)**

(74) Representative: **Fischer, Michael
Siemens AG
Postfach 22 16 34
80506 München (DE)**

(56) References cited:
**WO-A1-2010/089319**

• **ZHIHUA QI ET AL: "A novel method to reduce data acquisition time in differential phase contrast: computed tomography using compressed sensing", PROCEEDINGS OF SPIE, vol. 7258, 1 January 2009 (2009-01-01), pages 72584A-72584A-8, XP55013373, ISSN: 0277-786X, DOI: 10.1117/12.813861**

EP 2 652 708 B1

**Description**

The present invention relates to a method and a system for the recovery of an integrated image from a differential image by using constrained optimization.

It is well known that, differently from conventional visible light optics, the refractive index in X-ray optics is very close to and smaller than unity. In first approximation, for small and negligible anisotropy in the medium, the index of refraction characterizing the optical properties of a tissue can be expressed - including X-ray absorption - with its complex form: $n=1-\delta-i\beta$ where $\delta$ is the decrement of the real part of the refractive index, characterizing the phase shifting property, while the imaginary part $\beta$ describes the absorption property of the sample. In conventional absorption-based radiography, the X-ray phase shift information is usually not directly utilized for image reconstruction. However, at photon energies greater than 10 keV and for light materials (made up of low-Z elements), the phase shift term plays a more prominent role than the attenuation term because $\delta$ is typically three orders of magnitude larger than $\beta$. As a consequence, phase-contrast modalities can generate significantly greater image contrast compared to conventional, absorption-based imaging.

Furthermore, far from absorption edges, $\delta$ is inversely proportional to the square of the X-ray energy whilst $\beta$ decreases as the fourth power of energy. A significant consequence of this mechanism is that phase signals can be obtained with much lower dose deposition than absorption, a very important issue when radiation damage has to be taken into account such as in biological samples or in living systems.

Several approaches have been developed in order to record the phase signal. They can be classified as interferometric methods (with crystals), phase propagation methods, techniques based on an analyzer crystal, or on x-ray gratings. The described invention is in particular in context with the latter technique.

Zhihua Qi et al., "A novel method to reduce data acquisition time in differential phase contrast: computed tomography using compressed sensing", Proceedings of SPIE, vol. 7258, January 2009, discloses a compressed sensing (CS) based reconstruction framework for differential phase contrast CT which seeks to minimize the $L_1$ norm of a sparsified image $\psi X$ which meets, at the same time, a forward criterion $A\ \partial/\partial\rho\ X = Y$.

Grating based x-ray imaging setups essentially detect the deflections of x-rays in the object. Such deflections can be either caused by refraction on phase shift gradients in the object resulting in differential phase contrast (DPC) or by scattering on in-homogeneities in the sample resulting in the so-called dark-field image (DFI) contrast. The DPC image signal can be used to obtain phase contrast (PC) images by image processing routines.

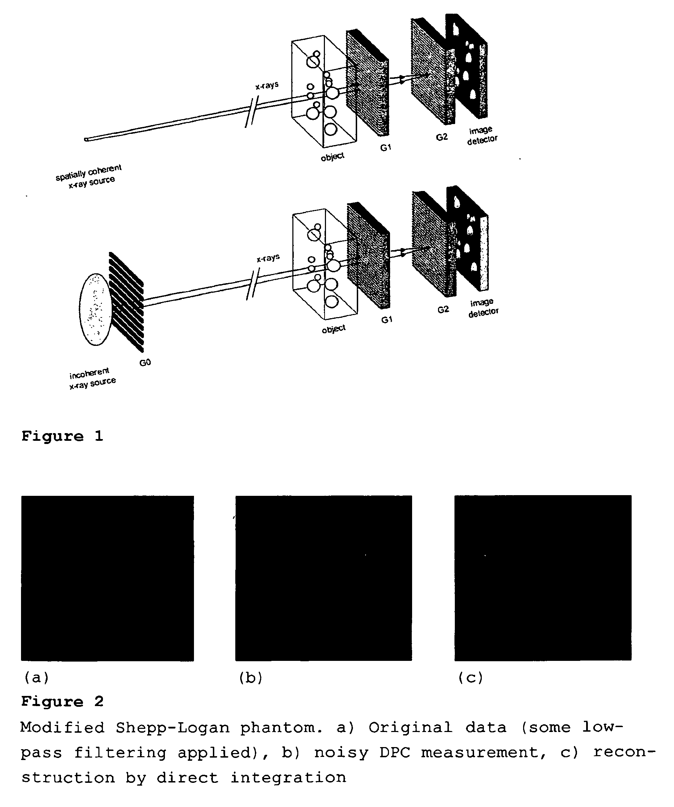

As shown in Figure 1, set-ups with two gratings (G1 and G2) or three gratings (G0, G1, and G2) can be applied to record the deflection of the x-rays. In the case of a two-grating set-up, the source needs to fulfill certain requirements regarding its spatial coherence, while in a three grating setup no spatial coherence is required. A G0 grating is required, when the source size is bigger than $p2*1/d$, where $p2$ is the period of G2, $1$ is the distance between the source and G1, and $d$ is the distance between G1 and G2. Therefore, the three grating set-up is suited for use with incoherent x-ray sources, in particular with x-ray tubes.

To separate the conventional attenuation contrast (AC) from the DPC and DFI contrast, a phase-stepping approach is applied. One of the gratings is displaced transversely to the incident beam whilst acquiring multiple images. The inten-sity signal at each pixel in the detector plane oscillates as a function of the displacement. The average value of the oscillation represents the attenuation contrast (AC). The phase of the oscillation can be directly linked to the first derivative of the wave-front phase profile and thus to the DPC signal. The amplitude of the oscillation depends on the scattering of x-rays in the object and thus yields the DFI signal.

For the (two or three) gratings, several approaches have been proposed and applied. The grating G0 (if required) is the one closest to the source. It usually consists of a transmission grating of absorbing lines with the period $p0$. It can be replaced by a source that emits radiation only from lines with the same period. The grating G1 is placed further downstream of the source. It consists of lines with a period $p1$. The grating G2 is the one most downstream of the setup. It usually consists of a transmission grating of absorbing lines with the period $p2$. It can be alternatively replaced by a detector system that has a grating-like sensitivity with the same period.

Two regimes of setups can be distinguished: in the so called "near field regime" and the "Talbot regime". In the "near field regime", the grating period $p$, grating distances $d$ and the x-ray wavelength $\lambda$ are chosen such, that diffraction effects are negligible. In this case, all gratings need to consist of absorbing lines. In the "Talbot regime", diffraction on the grating structures is significant. A sharp distinction between the two regimes is not easily given, as the exact criterion depends on the duty cycle of the grating structure, and whether the gratings are absorbing or phase shifting. E.g., for a grating with absorbing lines and a duty cycle of 0.5, the condition for the "near field regime" is $d \geq p^2/2\lambda$. Here, G1 should consist of grating lines that are either absorbing or, preferentially, phase shifting. Several amounts of phase shift are possible, preferentially $\pi/2$ or multiples thereof. The grating periods must be matched to the relative distances between the gratings. In case of setups in the "Talbot regime" the Talbot effect needs to be taken into account to obtain good contrast. The formulae for the grating periods and distances are known in the prior art.

The sample is mostly placed between G0 of G1 (or upstream of G1 in case of a two-grating set-up), however it can be advantageous to place it between G1 and G2.

The presented invention is relevant in all of the abovementioned cases, i.e. in the two- and three-grating case, in the case of the "nearfield regime" and the "Talbot regime", and for the sample placed upstream or downstream of G1.

In addition, the invention presented here also works in combination with scanning-based systems, for parallel and quasi parallel geometries using planar gratings or for compact fan-beam or cone-beam geometries using cylindrically or spherically curved gratings.

An imaging experiment with the abovementioned system is sensitive to X-ray refraction. The relation between the refractive angle $\alpha$ (relative to the optical axis) and the differential phase shift measured with the system is given by:

$$\Delta\varphi(x) = \frac{2\pi d}{p_2}\alpha(x) , \qquad (1)$$

where d is the inter-grating distance and $p_2$ is the period of G2. The x-axis corresponds to the phase stepping direction. Furthermore, the relationship between $\alpha(x)$ and the phase profile $\phi(x)$ of the wave after the sample is given by [12] :

$$\alpha(x) = \frac{\lambda}{2\pi}\frac{\partial\phi(x)}{\partial x} . \qquad (2)$$

Substituting Eq. (2) into Eq. (1) leads to

$$\Delta\varphi(x) = \frac{\lambda d}{p_2}\frac{\partial\phi(x)}{\partial x} . \qquad (3)$$

Therefore, $\Delta\varphi$ is proportional to the first derivative of $\phi(x)$. According to this equation, the reconstruction of $\phi(x)$ requires an integration of the DPC measurement in x direction,

$$\phi(x) = \frac{p_2}{\lambda d}\int\Delta\varphi(x)\, dx . \qquad (4)$$

In general, the integration of a noisy signal accumulates the noise errors and the noise variance. As a result, horizontal stripe artifacts with increasing amplitudes in the direction of integration appear. Figure 2 shows the integration of a noisy DPC image, generated from a modified Shepp-Logan phantom, where in Figure 2c, the stripe artifact are clearly visible.

It is therefore an objective of the present invention to provide a system and the method to improve the quality of the integrated phase contrast image.

This objective is achieved according to the present invention by the features of the independent claims 1 and 19. Preferred embodiments of the present invention are given in the dependent claims 2 to 18 and 20 to 36 resp.

The inventive system and the inventive method for the recovery of an integrated image from a differential image by using constrained optimization are comprising:

a) means for identifying an image that minimizes a cost function $\|Tf\|_{\ell_p}$ subject to a constraint $\|W(D_x f - \varphi)\|_{\ell_2}$, which is written as
minimize $\|Tf\|_{\ell_p}$
subject to: $\|W(D_x f - \varphi)\|_{\ell_2} \leq \varepsilon$'

b) wherein f is an image vector, T is a transform operator matrix, $D_x$ is a differentiation operator matrix, W a diagnoal matrix containing the reciprocal noise standard deviations, $\varphi$ is the measured image vector and $\varepsilon$ a boundary for the noise power. $\|...\|_{\ell_p}$ indicates the p-norm of a vector, where p is a positive number, preferably an integer in the interval [0,2].

A preferred embodiment of the present invention is achieved when the differential image is obtained from an arrangement for x-rays, in particular hard x-rays, for obtaining quantitative x-ray images from a sample comprising:

a. an X-ray source;

b. three or at least two gratings dubbed G0, G1 and G2 or G1 and G2 resp.;

c. a position-sensitive detector with spatially modulated detection sensitivity having a number of individual pixels;

d. means for recording the images of the detector;

e. means for evaluating the intensities for each pixel in a series of images in order to identify the characteristic of the object for each individual pixel as an absorption dominated pixel and/or a differential phase contrast dominated pixel and/or an x-ray scattering dominated pixel;

f. wherein the series of images is collected by continuously or stepwise rotating from 0 to n or 2n either the sample or the arrangement and the source relative to the sample.

Preferably, the system and the method can be operated either in the so-called "near field regime" or in the "Talbot-regime".

A preferred embodiment for the grating G1 provides for G1 as a line grating being either an absorption grating or a phase grating, wherein the phase grating is a low absorption grating but generating a considerable X-ray phase shift, the latter preferably of n/2 or multiples thereof.

Accordingly, the grating G2 can be realized as a line grating having a high X-ray absorption contrast with its period being the same as that of the self image of G1; G2 being placed in front of the detector with its lines parallel to those of G1.

The system and the method allow for a certain freedom of operation where an operation can be chosen to be either in parallel-beam, quasi parallel, fan-beam or cone-beam mode and G0, G1 and G2 have a corresponding planar, cylindrical or spherical shape resp. Accordingly, the operation can be chosen to be either in fullfield mode with two dimensional gratings or in scanning mode with one dimensional gratings.

For the setup of the system and the method, both types of operation can be defined as follows: a) for near-field-regime operation, the distance between the gratings is chosen freely within the regime, and b) for the Talbot-regime is chosen according to

$$D_{n,sph} = \frac{L \cdot D_n}{L - D_n} = \frac{L \cdot n \cdot p_1^2 / 2\eta^2\lambda}{L - n \cdot p_1^2 / 2\eta^2\lambda}$$

where n=1,3,5......, and $\eta = \begin{cases} 1 & \text{if the phase shift of } G_1 \text{ is } (2l-1)\frac{\pi}{2}, \quad p_2 = \frac{L + D_{n,sph}}{L} p_1 \\ 2 & \text{if the phase shift of } G_1 \text{ is } (2l-1)\pi, \quad p_2 = \frac{L + D_{n,sph}}{L}\frac{p_1}{2} \end{cases}$,

where $l$=1,2,3......, $D_n$ is an odd fractional Talbot distance when the parallel X-ray beam is used, while $D_{n,sph}$ is that when the fan or cone X-ray beam is used, $L$ is the distance between the source and the G1.

In order to benefit from the full advantages of the differential phase contrast approach, the system and the method are executed in a way that the phase stepping is performed by mechanical shift of one grating G0, G1 or G2 with respect to the others.

With respect to the grating structure, the grating structure may be advantageously manufactured by planar technology.

In order to separate the differential phase information in the image, such as a medical image from a CT, MRI or ultrasonic facility, the differential phase information may be obtained according to the International Published Patent Application No. WO 2012/000694.

In a further preferred embodiment of the present invention, the phase relation between G1 and G2 can correspond exactly to the value for which the intensity curve can be expanded by a first order Taylor series and the differential phase information can be obtained preferably according to the International Published Patent application No. WO 2010/089319.

During the solution of the mathematical cost function, the operator $D_x$ may be designed as a differentiation operator of any order in the phase stepping direction of the gratings. Accordingly, the transform operator $T$ may be chosen to be a differentiation operator of any order in the direction perpendicular to the stepping direction of the gratings. Further, the weighting operator $W$ may be chosen to be a diagonal weighting matrix containing the inverse standard deviation $1/\sigma_{DPC}$ of the DPC image in each pixel.

Preferably, the constrained optimization problem can be solved by recasting it to a second order cone program (SOCP). Alternatively, the constrained optimization problem may be solved by casting it to an unconstrained form possibly according to:

$$\text{minimize} \quad \|W(D_x f - \varphi)\|_{\ell_2}^2 + \sum_i \lambda_i \|T_i f\|_{\ell_{p_i}}^{p_i}$$

Alternatively, the unconstrained optimization problem may be solved by means of a Gradient descent or a (non-linear) Conjugate Gradient algorithm.

Thereby, the new invention addresses the problem of stripe artifacts upon direct integration of noisy DPC images. The fundamental idea is to suppress the variations in the vertical image direction by solving a constrained optimization problem. While maintaining consistency with the measured data, the phase image is retrieved by minimizing a cost function. Applied to the case of noisy DPC measurements, this forces the integration to generate lower variations in the image and therefore improve image quality.

Preferred example of the present invention are described hereinafter in more detail.

Hereafter, images, such as medical images or images from structure analysis or material testing and the like, are represented as vectors. An image vector $f(i)$ is obtained from the column-wise extraction of pixel values in an image $I(x,y)$

$$f(i) = I\left(\left\lfloor \frac{i}{n_y} \right\rfloor, i \bmod n_y\right) \tag{5}$$

where $n_x \times n_y$ is the image size. The size of an image vector is $1 \times n$ with $n = n_x \cdot n_y$. Image transformations are represented by operators (matrices) which can be applied to an image vector. Operator matrices are of the size $m \times n$, where $m$ is the size of the transformed vector. In most cases, $m = n$ holds.

The new method is based on a standard linear regression model, where the measurement of the DPC image is given by

$$\varphi = D_x \cdot \phi + w. \tag{6}$$

$D_x$ is the measurement operator modelling the differential measurement of $\phi$ and $w$ is a random vector modelling the noise in a DPC image. $D_x$ can be implemented with a finite difference transform of $\phi$ in x-direction.

Regarding the noise model, the noise variance in a pixel of a DPC image is given by

$$\sigma_{DPC}^2 \propto \frac{1}{NV^2}, \tag{7}$$

where $N$ is the number of photons and $V$ is the mean fringe visibility at this pixel. $N$ and $V$ can be calculated by using the AC and DFI images of the measurement.

For the phase retrieval from a DPC measurement, a constrained optimization problem is defined:

$$\text{minimize} \quad \left\| Tf \right\|_{\ell_p}$$

$$\text{subject to}: \quad \left\| W(D_x f - \varphi) \right\|_{\ell_2} \leq \varepsilon \tag{8}$$

In this optimization problem, $f$ is an image vector, $T$ is a transform operator, $\varphi$ is the measured DPC image vector and $\varepsilon$ is a boundary for the noise power. $W$ is a diagonal weighting matrix for taking into account the noise model, with $w_{i,i} = 1/\sigma_{DPC,i}$. $\|...\|_{\ell_p}$ indicates a $p$-norm of a vector, which is defined as

$$\left\| x \right\|_{\ell_p} = \left( \sum_i |x_i|^p \right)^{1/p}. \tag{9}$$

The problem expressed in (8) seeks for the minimal $p$-norm of the vector $Tf$ for any $f$ which meets the data consistency constraint $\|W(D_x f - \varphi)\|_{\ell_2} < \varepsilon$.

In the minimization term of (8), the image can be transformed into any linear transform domain represented by the matrix $T$ (e.g. Fourier transform, wavelet, finite differences, etc.). This makes the utilization of data constraints extremely flexible. In the case of DPC measurements, a finite differences transform

$$T = D_y, \tag{10}$$

is preferable, because the integrated image is distorted by high intensity variations (horizontal stripes) in the direction perpendicular to phase stepping.

There is a variety of possibilities to solve problem (8). In the convex case ($p \geq 1$), it can be recasted and solved as a second order cone program. An alternative is to recast (8) to an unconstrained form using a Lagrange multiplier,

$$\text{minimize} \quad \left\| W(D_x f - \varphi) \right\|_{\ell_2}^2 + \lambda \left\| Tf \right\|_{\ell_p}^p, \tag{11}$$

or in general, with an arbitrary amount of data constraints,

$$\text{minimize} \quad \left\| W(D_x f - \varphi) \right\|_{\ell_2}^2 + \sum_i \lambda_i \left\| T_i f \right\|_{\ell_{p_i}}^{p_i} \tag{12}$$

This problem formulation is also known as regularization, which has mainly been applied for the inversion of ill-posed problems. The Lagrangian multiplier (or regularization parameter) $\lambda_i$ controls the weighting of the regularization term $\left\| T_i f \right\|_{\ell_{p_i}}^{p_i}$ compared to the data conistency term $\left\| W(D_x f - \varphi) \right\|_{\ell_2}^2$. Problem (12) has the powerful property to allow any number of regularization terms, which is particularly useful if more a-priori knowledge about the object is available.

The choice of the norm parameter $p$ in the regularization term depends on the used transform operator $T$. A typical choice is $p=2$, since in this case, problems (11) / (12) are linear and an explicit solution exists. For the case $T = D_y$, the $\ell_2$-norm can lead to blurring and therefore reduce image resolution. On the other hand, the minimization of the $\ell_1$-norm (p=1) is well known to preserve edges in the image and is thus expected to yield a better image resolution than the $\ell_2$-norm [1]. An $\ell_1$-norm minimization leads to a non-linear optimization problem where no explicit solution exists.

Here, an iterative algorithm is used to solve the non-linear optimization problem. It is based on a non-linear Conjugate Gradients (NLCG) method, which is characterized by a fast convergence for the inversion of large-scale linear systems [2].

Advantageous features yet not listed in the claims are hereinafter:

- the grating structure is manufactured by planar technology; and/or

- the differential phase information is obtained according to International Published Patent Application No. WO 2012/000694; and/or

- the phase relation between G1 and G2 corresponds exactly to the value for which the intensity curve can be expanded by a first order Taylor series and the differential phase information is obtained preferably according to International Published Patent application No. WO 2010/089319.

A system that could be also subject to the claims is according to the present invention provided by a system for the recovery of an integrated image from a differential image by using constrained optimization; comprising:

a) means for identifying an image that minimizes a cost function $\|Tf\|_{\ell_p}$ subject to a constraint $\|W(D_x f - \varphi)\|_{\ell_2}$, which is written as

$$\text{minimize} \quad \|Tf\|_{\ell_p}$$

$$\text{subject to}: \quad \left\|W(D_x f - \varphi)\right\|_{\ell_2} \leq \varepsilon'$$

b) wherein $f$ is an image vector, $T$ is a transform operator matrix, $D_x$ is a differentiation operator matrix, W a diagonal matrix containing the reciprocal noise standard deviations, $\varphi$ is the measured image vector and $\varepsilon$ a boundary for the noise power. $\|...\|_{\ell_p}$ indicates the p-norm of a vector, where $p$ is a positive number, preferably an integer in the interval [0,2].

Further advantageous system attributes are alone or in any possible combination thereof:

- the differential image is obtained from an arrangement for x-rays, in particular hard x-rays, for obtaining quantitative x-ray images from a sample comprising:

    a. an X-ray source;

    b. three or at least two gratings dubbed G0, G1 and G2 or G1 and G2 resp.;

    c. a position-sensitive detector with spatially modulated detection sensitivity having a number of individual pixels;

    d. means for recording the images of the detector;

    e. means for evaluating the intensities for each pixel in a series of images in order to identify the characteristic of the object for each individual pixel as an absorption dominated pixel and/or a differential phase contrast dominated pixel and/or an x-ray scattering dominated pixel;

    f. wherein the series of images is collected by continuously or stepwise rotating from 0 to n or 2n either the sample or the arrangement and the source relative to the sample.

- the system being operated either in the so-called "near field regime" or in the "Talbot-regime";

- G1 is a line grating (G1) being either an absorption grating or a phase grating, wherein the phase grating is a low absorption grating but generating a considerable X-ray phase shift, the latter preferably of n/2 or multiples thereof;

- G2 is a line grating having a high X-ray absorption contrast with its period being the same as that of the self image of G1; G2 being placed in front of the detector with its lines parallel to those of G1;

- an operation is chosen to be either in parallel-beam, quasi parallel, fan-beam or cone-beam mode and G0, G1 and G2 have a corresponding planar, cylindrical or spherical shape resp;

- an operation is chosen to be either in fullfield mode with two dimensional gratings or in scanning mode with one dimensional gratings;

- for near-field-regime operation, the distance between the gratings is chosen freely within the regime, and for the Talbot-regime is chosen according to

$$D_{n,sph} = \frac{L \cdot D_n}{L - D_n} = \frac{L \cdot n \cdot p_1^2 / 2\eta^2 \lambda}{L - n \cdot p_1^2 / 2\eta^2 \lambda}$$

where n=1,3,5......, and $\eta = \begin{cases} 1 & \text{if the phase shift of } G_1 \text{ is } (2l-1)\dfrac{\pi}{2}, \quad p_2 = \dfrac{L + D_{n,sph}}{L} p_1 \\ 2 & \text{if the phase shift of } G_1 \text{ is } (2l-1)\pi, \quad p_2 = \dfrac{L + D_{n,sph}}{L} \dfrac{p_1}{2} \end{cases}$ ,

where $l$=1,2,3 ....... $D_n$ is an odd fractional Talbot distance when the parallel X-ray beam is used, while $D_{n,sph}$ is that when the fan or cone X-ray beam is used, $L$ is the distance between the source and the G1;

- phase stepping is performed by mechanical shift of one grating G0, G1 or G2 with respect to the others;

- the grating structure is manufactured by planar technology;

- the differential phase information is obtained according to International Published Patent Application No. WO 2012/000694;

- the phase relation between G1 and G2 corresponds exactly to the value for which the intensity curve can be expanded by a first order Taylor series and the differential phase information is obtained preferably according to International Published Patent application No. WO 2010/089319;

- the operator $D_x$ is a differentiation operator of any order in the phase stepping direction of the gratings;

- the transform operator $T$ is a differentiation operator of any order in the direction perpendicular to the stepping direction of the gratings;

- the weighting operator $W$ is a diagonal weighting matrix containing the inverse standard deviation $1/\sigma_{DPC}$ of the DPC image in each pixel;

- the constrained optimization problem is solved by recasting it to a second order cone program (SOCP);

- the constrained optimization problem is solved by casting it to an unconstrained form possibly according to:

$$\text{minimize} \quad \left\| W(D_x f - \varphi) \right\|_{\ell_2}^2 + \sum_i \lambda_i \left\| T_i f \right\|_{\ell_{p_i}}^{p_i} \ ;$$

- the unconstrained optimization problem is solved by means of a Gradient descent or a (non-linear) Conjugate Gradient algorithm.

**References**

[1] L. Rudin, S. Osher, and E. Fatemi, "Nonlinear total variation based noise removal algorithms," Phys. D Nonlinear Phenom. 60, 259-268 (1992)

[2] J. Nocedal and S. Wright, Numerical optimization (Springer Verlag, 1999)

**Claims**

1. A method for the recovery of an integrated image from a differential image by using constrained optimization; comprising the steps of:

   identifying an image that minimizes a cost function $\|Tf\|_{\ell_p}$ subject to the constraint $\|W(D_x f - \varphi\|_{\ell_2}$, which is written as

$$\text{minimize} \quad \|Tf\|_{\ell_p}$$

$$\text{subject to:} \quad \left\|W(D_x f - \varphi)\right\|_{\ell_2} \leq \varepsilon$$

   where $f$ is an image vector, $T$ is a transform operator matrix, $D_x$ is a differentiation operator matrix, W a diagnoal matrix containing the reciprocal noise standard deviations, $\varphi$ is the measured image vector and $\varepsilon$ a boundary for the noise power. $\|...\|_{\ell_p}$ indicates the $p$-norm of a vector, where $p$ is a positive number, preferably an integer in the interval [0,2].

2. A method according to claim 1, where the differential data is obtained from an arrangement for x-rays, in particular hard x-rays, for obtaining quantitative x-ray images from a sample comprising:

   a. an X-ray source;
   b. three or at least two gratings dubbed G0, G1 and G2 or G1 and G2.
   c. a position-sensitive detector with spatially modulated detection sensitivity having a number of individual pixels;
   d. means for recording the images of the detector;
   e. means for evaluating the intensities for each pixel in a series of images in order to identify the characteristic of the object for each individual pixel as an absorption dominated pixel and/or a differential phase contrast dominated pixel and/or an x-ray scattering dominated pixel;
   f. wherein the series of images is collected by continuously or stepwise rotating from 0 to $\pi$ or $2\pi$ either the sample or the arrangement and the source relative to the sample.

3. The method according to claim 1 or 2, operated either in the so-called "near field regime" or in the "Talbot-regime".

4. The method according to any of the preceding claims 1 to 3, wherein G1 is line grating (G1) either an absorption grating or a phase grating, that is a low absorption grating but generating a considerable X-ray phase shift, the latter preferably of n/2 or multiples thereof.

5. The method according to any of the preceding claims 1 to 4, wherein G2 is a line grating having a high X-ray absorption contrast with its period being the same as that of the self image of G1 and G2 is placed in front of the detector with its lines parallel to those of G1.

6. The method according to any of the preceding claims 1 to 5, wherein an operation is chosen to be either in parallel-beam, quasi parallel, fan-beam or cone-beam mode and G0, G1 and G2 have a corresponding planar, cylindrical or spherical shape resp.

7. The method according to any of the preceding claims 1 to 6, wherein an operation is chosen to be either in full-field mode with two dimensional gratings or in scanning mode with one dimensional gratings.

8. The method according to any of the preceding claims 1 to 7, wherein for near-field-regime operation, the distance between the gratings is chosen freely within the regime, and for the Talbot-regime is chosen according to

$$D_{n,sph} = \frac{L \cdot D_n}{L - D_n} = \frac{L \cdot n \cdot p_1^2 / 2\eta^2 \lambda}{L - n \cdot p_1^2 / 2\eta^2 \lambda}$$

where $n=1,3,5......$ , and $\eta = \begin{cases} 1 & \text{if the phase shift of } G_1 \text{ is } (2l-1)\frac{\pi}{2}, \quad p_2 = \frac{L + D_{n,sph}}{L} p_1 \\ 2 & \text{if the phase shift of } G_1 \text{ is } (2l-1)\pi, \quad p_2 = \frac{L + D_{n,sph}}{L} \frac{p_1}{2} \end{cases}$ ,

where $l=1,2,3......$, $D_n$ is an odd fractional Talbot distance when the parallel X-ray beam is used, while $D_{n,sph}$ is that when the fan or cone x-ray beam is used, $L$ is the distance between the source and the G1.

9. The method according to any of the preceding claims 1 to 8, wherein phase stepping is performed by mechanical shift of one grating G0, G1 or G2 with respect to the others.

10. The method according to any of the preceding claims, wherein the operator $D_x$ is a differentiation operator of any order in the phase stepping direction of the gratings.

11. The method according to any of the preceding claims, wherein the transform operator T is a differentiation operator of any order in the direction perpendicular to the stepping direction of the gratings.

12. The method according to any of the preceding claims, wherein the weighting operator $W$ is a diagonal weighting matrix containing the inverse standard deviation $1/\sigma_{DPC}$ of the DPC image in each pixel.

13. The method according to any of the preceding claims, wherein the constrained optimization problem is solved by recasting it to a second order cone program (SOCP).

14. The method according to any of the preceding claims, wherein the constrained optimization problem is solved by casting it to an unconstrained form possibly according to:

$$\text{minimize} \quad \left\| W(D_x f - \varphi) \right\|_{\ell_2}^2 + \sum_i \lambda_i \left\| T_i f \right\|_{\ell_{p_i}}^{p_i}$$

15. The method according to any of the preceding claims, wherein the unconstrained optimization problem is solved by means of a Gradient descent or a (non-linear) Conjugate Gradient algorithm or others.

## Patentansprüche

1. Verfahren zur Rückgewinnung eines integrierten Bildes aus einem Differenzbild unter Anwendung von Optimierung unter Nebenbedingungen,
   welches die folgenden Schritte umfasst:

   Identifizieren eines Bildes, welches eine Kostenfunktion $\|Tf\|_{l_p}$ unter der Nebenbedingung $\|W(D_x f - \varphi)\|_{l_2}$ optimiert, was geschrieben werden kann als
   minimiere $\|Tf\|_{l_p}$
   unter der Nebenbedingung: $\|W(D_x f - \varphi)\|_{l_2} \leq \varepsilon$
   wobei $f$ ein Bildvektor ist, $T$ eine Transformationsoperator-Matrix ist, $D_x$ eine Differentiationsoperator-Matrix ist, $W$ eine Diagonalmatrix ist, welche die reziproken Rausch-Standardabweichungen enthält, $\varphi$ der gemessene Bildvektor ist und $\varepsilon$ eine Schranke für die Rauschleistung ist. $\|...\|_{l_p}$ bezeichnet die $p$-Norm eines Vektors, wobei $p$ eine positive Zahl ist, vorzugsweise eine ganze Zahl im Intervall [0, 2].

2. Verfahren nach Anspruch 1, wobei die Differenzdaten von einer Anordnung für Röntgenstrahlen, insbesondere harte Röntgenstrahlen, zum Gewinnen quantitativer Röntgenbilder von einer Probe gewonnen werden, welche umfasst:

a. eine Röntgenquelle;

b. drei oder mindestens zwei Gitter, bezeichnet mit G0, G1 und G2 oder G1 und G2;

c. einen positionsempfindlichen Detektor mit räumlich modulierter Detektionsempfindlichkeit, der eine Anzahl von einzelnen Pixeln aufweist;

d. Mittel zum Aufzeichnen der Bilder des Detektors;

e. Mittel zum Auswerten der Intensitäten für jedes Pixel in einer Reihe von Bildern, um die Kenngröße des Objekts für jedes einzelne Pixel als ein durch Absorption dominiertes Pixel und/oder ein durch Differential-Phasenkontrast dominiertes Pixel und/oder ein durch Röntgenstreuung dominiertes Pixel zu identifizieren;

f. wobei die Reihe von Bildern gesammelt wird, indem entweder die Probe oder die Anordnung und die Quelle relativ zu der Probe kontinuierlich oder schrittweise von 0 bis $\pi$ oder $2\pi$ gedreht wird bzw. werden.

3. Verfahren nach Anspruch 1 oder 2, welches entweder in der sogenannten "Nahfeld-Betriebsart" oder in der "Talbot-Betriebsart" durchgeführt wird.

4. Verfahren nach einem der vorhergehenden Ansprüche 1 bis 3, wobei G1 ein Liniengitter (G1) ist, das entweder ein Absorptionsgitter oder ein Phasengitter ist, welches ein Gitter mit geringer Absorption ist, das jedoch eine beträchtliche Röntgenstrahlen-Phasenverschiebung erzeugt, wobei die Letztere vorzugsweise $\pi/2$ oder Vielfache davon beträgt.

5. Verfahren nach einem der vorhergehenden Ansprüche 1 bis 4, wobei G2 ein Liniengitter mit einem hohen Röntgenabsorptionskontrast ist, wobei seine Periode dieselbe ist wie diejenige des Selbstbildes von G1, und G2 vor dem Detektor angeordnet ist, wobei seine Linien parallel zu denjenigen von G1 sind.

6. Verfahren nach einem der vorhergehenden Ansprüche 1 bis 5, wobei ein Betrieb so gewählt wird, dass er entweder im Parallelstrahl-, Quasi-Parallelstrahl-, Fächerstrahl- oder Kegelstrahl-Modus erfolgt und G0, G1 und G2 eine entsprechende ebene, zylindrische bzw. kugelförmige Form aufweisen.

7. Verfahren nach einem der vorhergehenden Ansprüche 1 bis 6, wobei ein Betrieb so gewählt wird, dass er entweder im Vollfeld-Modus mit zweidimensionalen Gittern oder im Scan-Modus mit eindimensionalen Gittern erfolgt.

8. Verfahren nach einem der vorhergehenden Ansprüche 1 bis 7, wobei für einen Betrieb in der Nahfeld-Betriebsart der Abstand zwischen den Gittern innerhalb der Betriebsart frei gewählt wird und für die Talbot-Betriebsart gemäß

$$D_{n,sph} = \frac{L \cdot D_n}{L - D_n} = \frac{L \cdot n \cdot p_1^2 / 2\eta^2 \lambda}{L - n \cdot p_1^2 / 2\eta^2 \lambda},$$

gewählt wird,

wobei n = 1,3,5..., und gemäß

$$\eta = \begin{cases} 1, & \text{falls die Phasenverschiebung von } G_1 \quad (2l-1)\dfrac{\pi}{2} \quad \text{ist, } p_2 = \dfrac{L + D_{n,sph}}{L} p_1 \\ 2, & \text{falls die Phasenverschiebung von } G_1 \quad (2l-1)\pi \quad \text{ist, } p_2 = \dfrac{L + D_{n,sph}}{L} \dfrac{p_1}{2} \end{cases},$$

wobei $l$ = 1, 2, 3..., $D_n$ ein ungeradzahliger fraktionaler Talbot-Abstand ist, wenn das parallele Röntgenstrahlenbündel verwendet wird, während $D_{n,sph}$ dies ist, wenn das fächerförmige oder kegelförmige Röntgenstrahlenbündel verwendet wird, $L$ der Abstand zwischen der Quelle und G1 ist.

9. Verfahren nach einem der vorhergehenden Ansprüche 1 bis 8, wobei Phasenschieben durch mechanische Verschiebung eines Gitters G0, G1 oder G2 relativ zu den anderen durchgeführt wird.

10. Verfahren nach einem der vorhergehenden Ansprüche, wobei der Operator $D_x$ ein Differentiationsoperator beliebiger Ordnung in der Phasenschieberichtung der Gitter ist.

**11.** Verfahren nach einem der vorhergehenden Ansprüche, wobei der Transformationsoperator *T* ein Differentiationsoperator beliebiger Ordnung in der zur Schieberichtung der Gitter senkrechten Richtung ist.

**12.** Verfahren nach einem der vorhergehenden Ansprüche, wobei der Gewichtungsoperator W eine diagonale Wichtungsmatrix ist, welche die inverse Standardabweichung $1/\sigma_{DPC}$ des Differential-Phasenkontrast-Bildes in jedem Pixel enthält.

**13.** Verfahren nach einem der vorhergehenden Ansprüche, wobei das Optimierungsproblem unter Nebenbedingungen gelöst wird, indem es in ein konisches Programm zweiter Ordnung (SOCP) umformuliert wird.

**14.** Verfahren nach einem der vorhergehenden Ansprüche, wobei das Optimierungsproblem unter Nebenbedingungen gelöst wird, indem es in eine Form ohne Nebenbedingungen gebracht wird, möglicherweise gemäß:

$$\texttt{minimiere} \quad \left\|W(D_x f - \varphi)\right\|_{l_2}^2 + \sum_i \lambda_i \left\|T_i f\right\|_{l_{p_i}}^{p_i}$$

**15.** Verfahren nach einem der vorhergehenden Ansprüche, wobei das Optimierungsproblem ohne Nebenbedingungen mittels eines Gradientenabstiegsalgorithmus oder eines (nichtlinearen) Algorithmus der konjugierten Gradienten oder eines anderen Algorithmus gelöst wird.

**Revendications**

**1.** Procédé de récupération d'une image intégrée à partir d'une image différentielle en utilisant l'optimisation sous contrainte ;
comprenant les étapes consistant à :

identifier une image qui minimise une fonction de coût $\|Tf\|_{\ell_p}$, soumise à la contrainte $\|W(D_x f-\varphi)\|_{\ell_2}$, qui s'écrit sous la forme
minimiser $\|Tf\|_{\ell_p}$
soumise à : $\|W(D_x f-\varphi)\|_{\ell_2} \leq \varepsilon$
où *f* est un vecteur d'image, *T* est une matrice d'opérateurs de transformation, $D_x$ est une matrice d'opérateurs de différentiation, *W* une matrice diagonale contenant les écarts-types de bruit réciproque, φ est le vecteur d'image mesuré et ε une limite de la puissance de bruit, $\|...\|_{\ell_p}$ indique la norme *p* d'un vecteur, où *p* est un nombre positif, de préférence un nombre entier dans l'intervalle [0,2].

**2.** Procédé selon la revendication 1, dans lequel les données différentielles sont obtenues à partir d'un agencement de rayons X, en particulier de rayons X durs, pour obtenir des images de rayons X quantitatives à partir d'un échantillon, comprenant :

a. une source de rayons X ;
b. trois ou au moins deux réseaux doublés G0, G1 et G2 ou G1 et G2.
c. un détecteur sensible à la position avec une sensibilité de détection modulée spatialement ayant un nombre de pixels individuels ;
d. un moyen pour enregistrer les images du détecteur ;
e. un moyen pour évaluer les intensités de chaque pixel dans une série d'images afin d'identifier la caractéristique de l'objet pour chaque pixel individuel en tant que pixel dominé par une absorption et/ou pixel dominé par un contraste de phase différentielle et/ou pixel dominé par une dispersion de rayons X ;
f. dans lequel la série d'images est recueillie en tournant en continu ou par paliers de 0 à n ou 2n soit l'échantillon, soit l'agencement et la source par rapport à l'échantillon.

**3.** Procédé selon la revendication 1 ou 2, utilisé soit dans régime dit « de champ proche », soit dans le « régime de Talbot ».

**4.** Procédé selon l'une quelconque des revendications précédentes 1 à 3, dans lequel G1 est un réseau de lignes (G1), soit un réseau d'absorption, soit un réseau de phase, qui est un réseau de faible absorption, mais générant un déphasage de rayons X considérable, celui-ci étant de préférence de n /2 ou un multiple de celui-ci.

5. Procédé selon l'une quelconque des revendications précédentes 1 à 4, dans lequel G2 est un réseau de lignes ayant un contraste d'absorption de rayons X élevé, et sa période est la même que celle de l'auto-image de G1 et G2 est placé devant le détecteur avec ses lignes parallèles à celles de G1.

6. Procédé selon l'une quelconque des revendications précédentes 1 à 5, dans lequel un fonctionnement est choisi pour être soit en mode à faisceau parallèle, quasi parallèle, à faisceau en éventail ou à faisceau conique, et G0, G1 et G2 ont une forme planaire, cylindrique ou sphérique correspondante, respectivement.

7. Procédé selon l'une quelconque des revendications précédentes 1 à 6, dans lequel une opération est choisie pour être soit en mode plein champ avec des réseaux bidimensionnels, soit en mode balayage avec des réseaux unidimensionnels.

8. Procédé selon l'une quelconque des revendications précédentes 1 à 7, dans lequel pour un fonctionnement en régime en champ proche, la distance entre les réseaux est choisie librement au sein du régime, et pour le régime de Talbot, est choisie en fonction de

$$D_{n,sph} = \frac{L \cdot D_n}{L - D_n} = \frac{L \cdot n \cdot p_1^2 / 2\eta^2\lambda}{L - n \cdot p_1^2 / 2\eta^2\lambda}$$

où n=1, 3, 5 ...... et $n \begin{cases} 1 & \text{si le décalage de phase de G}_1 \text{ est } (2l-1)\frac{\pi}{2}, p_2 = \frac{L + D_{n,sph}}{L} p_1, \text{où } l = 1, 2, 3 ..., \\ 2 & \text{si le décalage de phase G}_1 \text{ est } (2l-1)\pi, p_2 = \frac{L + D_{n,sph}}{L} \frac{p_1}{2} \end{cases}$

$D_n$ est une distance de Talbot fractionnaire impaire lorsque le faisceau de rayons X parallèle est utilisé, tandis que $D_{n,sph}$ est celle lorsque le faisceau de rayons X en éventail ou conique est utilisé, L est la distance entre la source et G1.

9. Procédé selon l'une quelconque des revendications précédentes 1 à 8, dans lequel l'avance de phase est effectuée par décalage mécanique d'une grille G0, G1 ou G2 par rapport aux autres.

10. Procédé selon l'une quelconque des revendications précédentes, dans lequel l'opérateur $D_x$ est un opérateur de différenciation de n'importe quel ordre dans la direction d'avance de phase des réseaux.

11. Procédé selon l'une quelconque des revendications précédentes, dans lequel l'opérateur de transformation $T$ est un opérateur de différenciation de n'importe quel ordre dans la direction perpendiculaire à la direction d'avance des réseaux.

12. Procédé selon l'une quelconque des revendications précédentes, dans lequel l'opérateur de pondération W est une matrice de pondération diagonale contenant l'écart-type inverse $1/\sigma DPC$ de l'image DPC dans chaque pixel.

13. Procédé selon l'une quelconque des revendications précédentes, dans lequel le problème d'optimisation sous contrainte est résolu par sa refonte en un second programme conique du second ordre (SOCP).

14. Procédé selon l'une quelconque des revendications précédentes, dans lequel le problème d'optimisation sous contrainte est résolu par sa refonte en une forme non contrainte, éventuellement en fonction de : minimiser

$$\|W(D_x f - \varphi)\|_{\ell_2}^2 + \sum_i \lambda_i \|T_i f\|_{\ell_{p_i}}^{p_i}$$

15. Procédé selon l'une quelconque des revendications précédentes, dans lequel le problème d'optimisation non contrainte est résolu au moyen d'une descente de gradient ou d'un algorithme de gradient conjugué (non linéaire) ou autres.

**Figure 1**

(a)                    (b)                    (c)

**Figure 2**

Modified Shepp-Logan phantom. a) Original data (some low-pass filtering applied), b) noisy DPC measurement, c) reconstruction by direct integration

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012000694 A

- WO 2010089319 A

**Non-patent literature cited in the description**

- **ZHIHUA QI et al.** A novel method to reduce data acquisition time in differential phase contrast: computed tomography using compressed sensing. *Proceedings of SPIE,* January 2009, vol. 7258

- **L. RUDIN ; S. OSHER ; E. FATEMI.** Nonlinear total variation based noise removal algorithms. *Phys. D Nonlinear Phenom,* 1992, vol. 60, 259-268
- **J. NOCEDAL ; S. WRIGHT.** Numerical optimization. Springer Verlag, 1999